# FASCICULE DE BREVET EUROPEEN

(11) **EP 3 880 263 B1**
(45) Date de publication et mention de la délivrance du brevet: **15.02.2023**
(21) Numéro de dépôt: 19801886.3
(22) Date de dépôt: 14.11.2019
(51) Int. Cl.: A61L 27/44, A61L 27/54, A61L 27/56

(54) **IMPLANT A POROSITE CONTROLEE EN UN MATERIAU HYBRIDE DOPE EN NUTRIMENT OSTEOINDUCTEUR**
IMPLANTAT MIT KONTROLLIERTER POROSITÄT AUS EINEM MIT OSTEOINDUKTIVEM NÄHRSTOFF DOTIERTEN HYBRIDMATERIAL
IMPLANT WITH CONTROLLED POROSITY MADE FROM A HYBRID MATERIAL DOPED WITH OSTEOINDUCTIVE NUTRIENT

(30) Priorité: 15.11.2018 FR 1860557
(43) Date de publication de la demande: 22.09.2021
(73) Titulaire: Universite Clermont Auvergne, 63000 Clermont Ferrand (FR); Centre national de la recherche scientifique, 75016 Paris (FR); Institut national de recherche pour l'agriculture, l'alimentation et l'environnement, 75007 Paris (FR)
(72) Inventeur: LAO, Jonathan, Claude, Alexandre, 63000 Veyre-Monton (FR); BOSSARD, Cédric, 63100 CLERMONT-FERRAND (FR); JALLOT, Edouard, Daniel, Albert, 63360 SAINT-BEAUZIRE (FR); WITTRANT, Yohann, 63450 CHANONAT (FR); GRANEL, Henri, 63130 ROYAT (FR)
(74) Mandataire: Gevers & Orès
(86) Numéro de dépôt international: PCT/EP2019/081383
(87) Numéro de publication internationale: WO 2020/099588

(56) Documents cités:
- WO-A1-2014/195863
- WO-A1-2015/172101
- JOAQUIN RODENAS-ROCHINA ET AL: "Comparative study of PCL-HAp and PCL-bioglass composite scaffolds for bone tissue engineering", JOURNAL OF MATERIALS SCIENCE: MATERIALS IN MEDICINE, vol. 24, no. 5, 1 mai 2013 (2013-05-01), pages 1293-1308, XP055132431, ISSN: 0957-4530, DOI: 10.1007/s10856-013-4878-5

## Description

### Domaine Technique

L'invention concerne un matériau d'implant pour le comblement de défauts osseux, la régénération osseuse et l'ingénierie tissulaire de l'os, un implant comprenant ce matériau et un procédé de fabrication d'un tel matériau d'implant.

### Arrière-plan technique

Le vieillissement global de la population et les troubles du système ostéo-articulaire qui l'accompagnent rendent nécessaire le développement de matériaux de substitution des tissus osseux de haute performance. 18 milliards d'euros de frais de santé sont en effet dépensés chaque année en France pour les maladies du système ostéo-articulaire et dentaires, les troubles musculo-squelettiques sont les pathologies professionnelles les plus répandues dans les pays industrialisés, tandis que l'ostéoporose se développe chez les patients âgés ; ces faits dessinent les contours d'un enjeu sociétal et économique majeur et expliquent la demande croissante en biomatériaux, implants à durée de vie accrue capables de combler les pertes osseuses.

Le recours aux greffes étant limité, et les matériaux d'origine animale pouvant poser des problèmes de biocompatibilité ou des risques d'infection, les efforts de recherche visent à élaborer des biomatériaux synthétiques capables de promouvoir la régénération osseuse.

On parle dans ce cas d'implants bioactifs : le matériau implanté n'est pas simplement destiné à combler de manière passive une perte osseuse en restant le plus inerte possible, mais au contraire il doit stimuler et participer activement au mécanisme de régénération osseuse. Ceci est particulièrement important dans le cas de larges défauts osseux, pour lesquels le mécanisme d'autoréparation ne fonctionne plus.

Actuellement les principaux matériaux bioactifs utilisés comme substituts osseux sont les « céramiques » bioactives, telles que les phosphates de calcium, et les verres bioactifs, également appelés « bioverres ».

Les premières céramiques bioactives ont été développées par L.L. Hench (L.L. Hench et al., J. Biomed. Mater. Res. 1971, 2, 117-141 ; L.L. Hench et al., J. Biomed. Mater. Res. 1973, 7, 25-42).

Les premiers verres bioactifs ont été préparés à partir de SiO₂, de P₂O₅, de CaO et de Na₂O. Les oxydes de silicium et de phosphore sont des formateurs de réseau qui participent à la cohésion du réseau vitreux. Les alcalins et alcaline terreux comme le sodium et le calcium ne présentent pas cette capacité et viennent modifier le réseau vitreux en y introduisant des ruptures de chaînes qui sont à l'origine de la faible température de fusion de ces verres associée à un désordre structural accru. Leur présence a pour conséquence une plus grande réactivité des verres bioactifs à travers notamment leur corrosion dans un environnement aqueux. Cette réactivité permet la formation d'hydroxyapatite en milieu physiologique et favorise donc la reconstruction osseuse.

Le bioverre qui a été le plus étudié est un verre sodo-silico-phosphocalcique dit Bioglass^{®} ou Bioverre de Hench. Sa composition de base est 45% SiO₂ - 24,5% CaO - 24,5% Na₂O - 6% P₂O₅, en masse par rapport à la masse totale de la composition. Les propriétés bioactives remarquables de ce matériau ne sont plus à démontrer. Le Bioglass^{®} reste à l'heure actuelle un des matériaux bioactifs (induisant une réponse spécifique des cellules) les plus intéressants.

De nombreux développements ont été faits dans le domaine des verres bioactifs depuis leur découverte (M. Vallet-Regi et al., Eur. J. Inorg. Chem. 2003, 1029-1042), tels que l'incorporation de différents atomes ou l'incorporation de principes actifs. Les compositions des verres bioactifs ont été optimisées de façon à favoriser la prolifération des ostéoblastes et la formation de tissus osseux (WO 02/04606). L'incorporation d'argent a été proposée notamment pour conférer des propriétés antibactériennes aux verres bioactifs (WO 00/76486).

La demande WO 2009/027594 décrit, elle, un verre bioactif dans lequel le strontium est introduit en des quantités comprises entre 0,1 à 10% du poids total du verre bioactif.

Ces matériaux bioactifs présentent comme caractéristique d'être tout à la fois biocompatibles, capables de se lier spontanément aux tissus osseux, de promouvoir l'adhésion des cellules osseuses et, enfin d'être biorésorbables, étant progressivement remplacés par du tissu osseux néoformé à mesure que la repousse osseuse avance.

Cependant, malgré cet ensemble de caractéristiques très satisfaisantes, la fragilité de ces matériaux en limite les applications : en effet, bien que leur rigidité soit souvent supérieure à celle de l'os, leur manque de souplesse et de ténacité font que les matériaux bioactifs ne peuvent être implantés en des sites soumis à des contraintes mécaniques cycliques.

Pour pallier ce défaut, la demande de brevet EP 3003414 décrit un matériau d'implant pour le comblement de défauts osseux, la régénération osseuse et l'ingénierie tissulaire de l'os, comprenant un matériau hybride à base d'un verre bioactif M à base de SiO₂ et CaO, optionnellement contenant P₂O₅ et/ou optionnellement dopé au strontium, et d'un polymère biodégradable P choisi parmi :
- les polysaccharides biorésorbables, de préférence choisis parmi le dextran, l'acide hyaluronique, l'agar, le chitosane, l'acide alginique, l'alginate de sodium ou de potassium, le galactomannane, le carraghénane, la pectine,
- les polyesters biorésorbables, de préférence l'alcool polyvinylique ou le poly(acide lactique),
- les polymères synthétiques biodégradables, de préférence un polyéthylène glycol, ou le poly(caprolactone) (abrégé ci-après par PCL), et
- les protéines, de préférence la gélatine ou le collagène.

Ce matériau consiste en une matrice comprenant le matériau hybride, cette matrice ayant au moins 70% en nombre de pores ayant la forme de sphères ou de polyèdres sphériques s'inscrivant dans une sphère, le diamètre des sphères étant compris entre 100 et 900 µm, de préférence entre 200 et 800 µm, bornes incluses, avec un écart entre le diamètre de la sphère la plus petite ou la plus grande étant au plus de 70%, de préférence au plus de 50%, plus préférablement au plus de 30%, par rapport au diamètre moyen arithmétique de l'ensemble des sphères de l'implant et les interconnexions entre les pores ayant leur plus petite dimension comprise entre 25 µm et 250 µm, bornes incluses, au moins 70% en nombre de ces pores ayant au moins une interconnexion avec un autre pore.

Les implants obtenus à partir de ce matériau ont des propriétés mécaniques proches du tissu osseux, et une morphologie spécifique s'inspirant de l'os trabéculaire, à savoir une structure hautement poreuse constituée d'un réseau tridimensionnel de macropores interconnectés de plusieurs centaines de microns. En effet, dans le cas de larges défauts osseux, les cellules de l'os ont besoin d'une matrice "support" extracellulaire capable de guider et de stimuler l'adhésion, la prolifération, la différenciation cellulaire, tout en étant compatible avec les processus de vascularisation et d'invasion tissulaire.

Une telle structure macroporeuse est également requise pour les nouvelles applications envisagées en ingénierie tissulaire de l'os: il s'agit, à partir de cellules prélevées chez le patient, de fabriquer en laboratoire du tissu osseux nouveau que l'on pourra ré-implanter a posteriori chez le patient. Pour être conduite de façon optimale, cette culture de tissu doit là aussi reposer sur des supports tridimensionnels poreux permettant une bonne adhésion cellulaire, la différenciation en cellules matures ainsi que la fabrication du tissu et en particulier la biominéralisation.

Bien que d'excellents résultats en termes de régénération osseuse aient été obtenus in vivo avec des implants en le matériau hybride verre bioactif polymère biodégradable décrit dans la demande de brevet EP 3003414, le caractère ostéoinducteur de ce matériau pourrait ne pas suffire pour stimuler la repousse des tissus osseux chez certains patients souffrant de troubles du métabolisme osseux, comme les patients âgés ou souffrant d'ostéoporose.

Des nutriments ostéoinducteurs pour stimuler la repousse des tissus osseux sont connus.

Des exemples de tels nutriments ostéoinducteurs sont la vitamine D (vitamine D2 (ergocalciférol), la vitamine D3 (cholicalciférol)), les dérivés et précurseurs de vitamine D, la vitamine K1, la vitamine K2, les acides gras oméga-3, l'acide punicique, l'acide α-lipoïque, les anthocyanines, les polyphénols, les flavonols, les procyanidines, le tyrosol, l'oleuropéïne, la naringénine, la punicalagine, l'acide ellagique, la phycocyanine, et le collagène hydrolysé.

Cependant, ces nutriments ostéoinducteurs sont généralement utilisés comme compléments alimentaires, en particulier les polyphénols, les composés phénoliques, et plus particulièrement la fisétine et l'hydroxytyrosol.

La fisétine est un polyphénol naturellement présent dans de nombreux fruits et légumes tels que la fraise, la pomme et le concombre, et l'hydroxytyrosol est un composé phénolique naturellement présent dans l'olive.

Ces deux composés sont reconnus pour leurs propriétés antioxydantes et il a été prouvé qu'une alimentation riche en fisétine ou en hydroxytyrosol renforce les os (Léotoing et al, The flavanoid fisetin promotes osteoblasts differentiation through Runx2 transcriptional activity, Mol. Nutr. Food Res. 58(6), 1239-1248, 2014, Mével, Elsa thèse de doctorat en sciences de la vie et de la santé, Biomolécules, pharmacologie, thérapeutique, Université de Nantes, *Utilisation des procyanidines et de l'hydroxytyrosol dans la prévention nutritionnelle de l'arthrose,* 2015).

Cependant, ces nutriments ostéoinducteurs utilisés en tant que compléments alimentaires souffrent de plusieurs désavantages ;
Tout d'abord, la biodisponibilité : le nutriment ingéré doit passer l'épithélium digestif et seulement une fraction de la quantité ingérée atteint la circulation sanguine.

Ensuite, l'administration par voie orale conduit à une dilution du nutriment dans la globalité de la circulation sanguine.

La faible biodisponibilité et la dilution du nutriment ingéré impliquent des doses conséquentes de nutriment à administrer par voie orale.

Des implants en polymère biodégradable ou encore en polysaccharides dans le réseau desquels un médicament, une molécule biologique, un facteur de croissance, etc., a été introduit sont connus.

Par ailleurs, des implants sur lesquels un médicament ou autre composé est adsorbé pour une délivrance directement au site d'intérêt sont connus.

Mais, l'adsorption ne permet pas d'obtenir une libération lente et prolongée du composé ou du médicament. La libération se produit avec un phénomène de « burst », c'est-à-dire une libération brusque et courte.

Des implants en polymère biodégradable ou encore en polysaccharides dans le réseau desquels un médicament, une molécule biologique, un facteur de croissance, etc., a été introduit sont connus.

Cependant, ces implants ne reproduisent pas la structure de l'os : ils ne comportent pas de partie(s) inorganique(s).

Dans ce contexte, l'invention vise à fournir un matériau d'implant en un matériau hybride polymère biodégradable-verre bioactif libérant de façon prolongée et régulière dans le temps un nutriment ostéoinducteur habituellement utilisé par voie orale.

### Résumé de l'invention

A cet effet, l'invention propose un matériau d'implant tel que défini dans la revendication 1.

De préférence, dans le matériau d'implant de l'invention, le rapport en poids polymère biodégradable P/ verre bioactif M est compris entre 20/80 et 80/20, bornes incluses et le nutriment ostéoinducteur N est présent en une quantité comprise entre 0,1 et 10 %, de préférence entre 0,1 et 5%, le plus préférablement 1%, en masse par rapport à la masse totale (verre bioactif M + polymère biodégradable P + nutriment N).

Encore de préférence, le matériau d'implant de l'invention consiste en une matrice comprenant le matériau hybride dopé en un nutriment ostéoinducteur N, cette matrice ayant au moins 70 % en nombre de pores ayant la forme de sphères ou de polyèdres s'inscrivant dans une sphère, le diamètre des sphères étant compris entre 100 et 900 µm, de préférence entre 200 et 800 µm, bornes incluses, avec un écart entre le diamètre de la sphère la plus petite ou la plus grande étant au plus de 70%, de préférence au plus de 50%, plus préférablement au plus de 30%, par rapport au diamètre moyen arithmétique de l'ensemble des sphères de l'implant et les interconnexions entre les pores ayant leur plus petite dimension comprise entre 25 µm et 250 µm, bornes incluses, au moins 70% en nombre de pores ayant au moins une interconnexion avec au moins un autre pore.

Dans un premier mode de réalisation préféré du matériau d'implant de l'invention, le matériau hybride dopé en un nutriment ostéoinducteur comprend 30% en masse de verre bioactif M à base de SiO₂ et CaO, par rapport à la masse totale (verre bioactif M + polymère biodégradable P + nutriment N), 69% en masse de poly(caprolactone), par rapport à la masse totale (verre bioactif M + polymère biodégradable P + nutriment N), et 1% en masse de fisétine et/ou d'hydroxytyrosol, par rapport à la masse totale (verre bioactif M + polymère biodégradable P + nutriment N).

Dans un second mode de réalisation préféré du matériau d'implant de l'invention, le matériau hybride dopé en un nutriment ostéoinducteur comprend 40% en masse de verre bioactif M à base de SiO₂ et CaO, par rapport à la masse totale (verre bioactif M + polymère biodégradable P + nutriment N), 59% en masse de poly(caprolactone), par rapport à la masse totale (verre bioactif M + polymère biodégradable P + nutriment N), et 1% en masse de fisétine et/ou d'hydroxytyrosol, par rapport à la masse totale (verre bioactif M + polymère biodégradable P + nutriment N).

Dans tous ses modes de réalisation, de préférence, le matériau d'implant de l'invention consiste en une matrice comprenant le matériau hybride dopé en un nutriment ostéoinducteur N, cette matrice ayant au moins 70 % en nombre de pores ayant la forme de sphères ou de polyèdres s'inscrivant dans une sphère, le diamètre des sphères étant compris entre 100 et 900 µm, de préférence entre 200 et 800 µm, bornes incluses, avec un écart entre le diamètre de la sphère la plus petite ou la plus grande étant au plus de 70%, de préférence au plus de 50%, plus préférablement au plus de 30%, par rapport au diamètre moyen arithmétique de l'ensemble des sphères de l'implant et les interconnexions entre les pores ayant leur plus petite dimension comprise entre 25 µm et 250 µm, bornes incluses, au moins 70% en nombre de pores ayant au moins une interconnexion avec au moins un autre pore.

L'invention propose également un implant en un matériau hybride pour le comblement de défauts osseux, la régénération osseuse et l'ingénierie tissulaire de l'os, qui comprend un matériau d'implant selon l'invention.

Le matériau d'implant de l'invention peut être obtenu par le procédé selon la revendication 5 qui est aussi un objet de l'invention.
- le matériau de l'agent porogène A est choisi parmi les polymères biodégradables insolubles dans le au moins un solvant S1 et dans le au moins un solvant S2. Le matériau de l'agent porogène A est soluble dans le au moins un solvant S. Le matériau de l'agent porogène A est de préférence choisi parmi les polyméthacrylates d'alkyle en C₁ à C₄, de préférence le polyméthacrylate de méthyle ou le polyméthacrylate de butyle, le polyuréthane, l'acide polyglycolique, les différentes formes d'acides polylactiques, les copolymères d'acides lactique-coglycoliques, le poly(caprolactone), le polypropylène fumarate, la paraffine et le naphtalène, ou l'acrylonitrile butadiène styrène (ABS),
le matériau de l'agent porogène A étant différent du polymère biodégradable P.

Dans ce procédé, de préférence :

Egalement de préférence, le rapport en poids polymère biodégradable P/ verre bioactif M est compris entre 20/80 et 80/20, bornes incluses et le nutriment ostéoinducteur N est présent en une quantité comprise entre 0,1 et 10 %, de préférence entre 0,1 et 5%, le plus préférablement 1%, en masse par rapport à la masse totale du matériau obtenu à l'étape k).

Dans un mode de réalisation préféré du procédé de l'invention, le verre bioactif M est un verre à base de SiO₂ et de CaO, le polymère biodégradable P est le poly(caprolactone), le nutriment ostéoinducteur N est la fisétine et/ou l'hydroxytyrosol, le matériau des microsphères d'agent porogène A est la paraffine, le solvant S est le cyclohexane, et le solvant S1 est identique au solvant S2 et est le tétrahydrofurane (THF).

### Brève description des figures

L'invention sera mieux comprise et d'autres caractéristiques et avantages de celle-ci apparaîtront plus clairement à la lecture de la description explicative qui suit et qui est faite en référence aux figures annexées dans lesquelles :
[Fig 1] la figure 1 représente une photographie prise au microscope électronique à balayage de l'implant obtenu à l'exemple 1 (comparatif), dont la matrice est constituée d'un matériau hybride poly(caprolactone)-verre bioactif non dopé à un grandissement de ×70,
[Fig 2] la figure 2 représente une photographie prise au microscope électronique à balayage, à un grandissement de ×70 d'une coupe du matériau d'implant obtenu à l'exemple 2, constitué d'un matériau hybride poly(caprolactone)-verre bioactif dopé avec de la fisétine,
[Fig 3] la figure 3 représente sous forme d'histogramme les résultats de l'évaluation in vitro des implants selon l'invention obtenus aux exemples 2 et 3 en comparaison à l'implant de l'art antérieur obtenu à l'exemple comparatif (exemple 1). La différentiation d'ostéoblastes primaires de rats est ici évaluée grâce à la mesure de l'activité enzymatique de la phosphatase alcaline (ALP) pour témoigner de l'effet du dopage sur les implants (exemple 2 et 3),
[Fig 4] la figure 4 montre sous forme de graphe le niveau de viabilité/prolifération cellulaire des ostéoblastes primaires de rat, après 7 jours de culture en présence de :
   - un contrôle, sans matériau (taux de 100%)
   - le matériau obtenu à l'exemple 1,et
   - le matériau obtenu à l'exemple 2,
[Fig 5] la figure 5 montre des images en microscopie électronique à balayage d'ostéoblastes primaires de rats (RPO) cultivés sur :
   - Photographies du haut : des tranches d'os cortical humain à 2 grandissements différents,
   - Photographies du milieu : des disques de BG-PCL (le matériau obtenu à l'exemple 1),et
   - Photographies du bas : les disques de BG-PCL-Fis (le matériau obtenu à l'exemple 2),
[Fig 6] la figure 6 montre les résultats de l'implantation des matériaux dans un modèle de défauts critiques en calvaria de souris (craniotomie). Ces résultats ont été obtenus par micro-tomographie par rayon X et permettent de distinguer :
   - les défauts osseux des animaux « contrôle » laissés vides chez,
   - les défauts osseux remplis avec BG-PCL (le matériau obtenu à l'exemple 1), et
   - les défauts osseux remplis avec BG-PCL-Fis (le matériau obtenu à l'exemple 2),
[Fig 7] la figure 7 représente l'évolution de la quantification de la régénération osseuse après implantation en défaut critique en calvaria de souris. Les résultats sont exprimés en % de formation d'os nouveau par rapport au jour d'implantation (J0) pour :
   - les défauts osseux de contrôle laissés vides chez,
   - les défauts osseux remplis avec BG-PCL (le matériau obtenu à l'exemple 1), et
   les défauts osseux remplis avec BG-PCL-Fis (le matériau obtenu à l'exemple 2).

### Description détaillée de l'invention

Dans ce qui précède et ce qui suit, les termes suivants ont les définitions suivantes :
- "interconnexion(s) entre pores" : ouverture(s) permettant le passage d'un pore à l'autre,
- "milieu aqueux" : tout milieu liquide contenant de l'eau, ou eau seule,
- "biodégradable" : dégradable dans un liquide physiologique, par exemple une solution saline tamponnée (SBF),
- "biorésorbable" : éliminable dans un milieu physiologique contenant des cellules biologiques,
- "diamètre moyen arithmétique de l'ensemble des pores" : somme des diamètres des pores / nombre de pores,
- "pore sphérique" ou "sphère" : pore ou sphère dont le rapport du plus petit diamètre sur le plus grand diamètre est de 0,9 ± 0,1,
- "polyèdre s'inscrivant dans une sphère" : polyèdre s'inscrivant dans une sphère ayant en tous points le même diamètre, les différences entre les différents diamètres du polyèdre s'inscrivant dans cette sphère étant d'au plus ± 15% du diamètre de la sphère dans laquelle ils s'inscrivent,
- "empilement compact de microsphères d'agent porogène A" : empilement de microsphères d'agent porogène A dans lequel :
   au moins 70% en nombre, de préférence plus de 95% en nombre de microsphères sont en contact les unes avec les autres, et restent en contact les unes avec les autres lorsque le mélange agent porogène A et hybride polymère biodégradable P-verre bioactif M-nutriment N est dans le moule, et lorsque l'empilement de microsphères est recouvert et infiltré avec le mélange hybride verre bioactif M-polymère biodégradable P-nutriment N.

On peut obtenir un tel empilement compact de microsphères d'agent porogène A par centrifugation du mélange microsphères d'agent porogène A et hybride polymère biodégradable P-verre bioactif M-nutriment ostéoinducteur N ou encore en appliquant une pression négative (vide) ou positive (supérieure à la pression atmosphérique) sur le mélange microsphères d'agent porogène A et hybride polymère biodégradable P-verre bioactif M-nutriment ostéoinducteur N introduit dans le moule, avant et pendant la gélification de ce mélange,
- « matériau hybride » : matériau comprenant une phase polymère et une phase verre bioactif, la phase verre bioactif consistant en des chaînes de nanoparticules de verre bioactif ( en contraste à des nanoparticules) qui se relient pour former un réseau tridimensionnel et qui sont entremêlées aux chaînes du polymère, au moins l'une des phases étant circonscrite à des domaines de taille inférieure à la centaine de nm, conférant ainsi au matériau hybride la propriété de réagir comme une seule phase au-delà de l'échelle moléculaire,
- « matériau hybride dopé » : matériau comprenant une phase polymère, une phase verre bioactif, et un nutriment ostéoinducteur, la phase verre bioactif consistant en des chaînes de nanoparticules de verre bioactif qui se relient pour former un réseau tridimensionnel, et qui sont entremêlées aux chaînes du polymère, au moins l'une des phases étant circonscrite à des domaines de taille inférieure à la centaine de nm, conférant ainsi au matériau hybride la propriété de réagir comme une seule phase au-delà de l'échelle moléculaire, le nutriment ostéoinducteur étant intégré dans le réseau tridimensionnel des chaînes de nanoparticules de verre bioactif et/ou les chaînes du polymère.

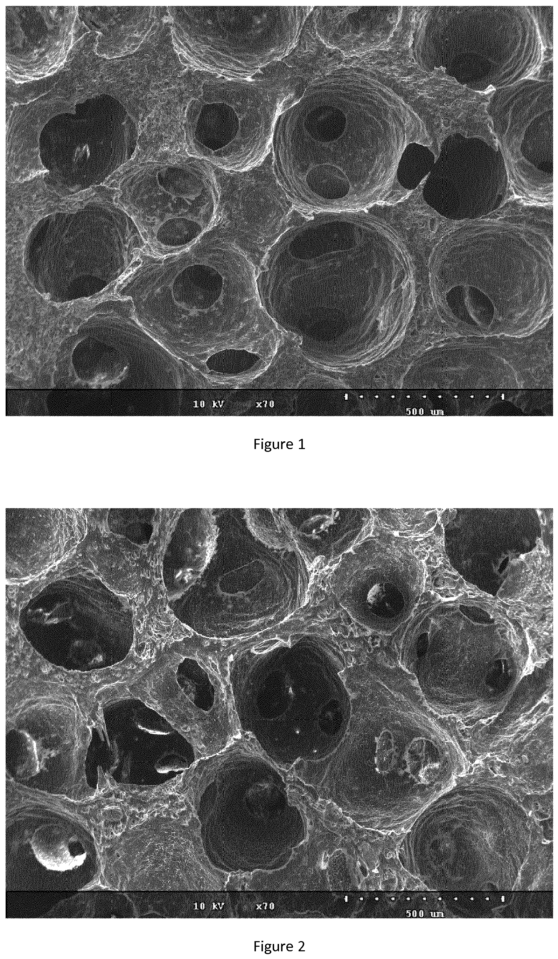

Le matériau d'implant pour le comblement de défauts osseux, la régénération osseuse et l'ingénierie tissulaire de l'os sera décrit en relation avec les figures 1 et 2.

Le matériau d'implant de l'invention comprend une matrice en un matériau hybride verre bioactif M-polymère biodégradable P dopé avec un nutriment ostéoinducteur N introduit dans le réseau du matériau hybride.

Cette configuration permet d'obtenir une libération du nutriment ostéoinducteur N directement au site d'intérêt, de manière prolongée et régulière, c'est-à-dire sans effet de « burst ». car le nutriment ostéoinducteur N est réparti uniformément dans la masse du matériau hybride et est libéré à mesure que ce dernier se dégrade.

Le nutriment ostéoinducteur N n'est pas ici dégradé et la quantité totale de ce nutriment ostéoinducteur N parvient donc au site d'intérêt.

Dans un mode de réalisation préféré, la matrice du matériau d'implant de l'invention a une porosité particulière qui est celle montrée en figure 2.

Comme on le voit en comparant les figures 1 et 2, le matériau d'implant de l'invention comprenant une matrice en un matériau hybride polymère biodégradable-verre bioactif dopé avec un nutriment ostéoinducteur représenté en figure 2 a une morphologie identique à celle du matériau d'implant en matériau hybride polymère biodégradable-verre bioactif non dopé de l'art antérieur représenté en figure 1.

Il comprend une matrice en un matériau qui comprend une partie organique et une partie inorganique et un nutriment ostéoinducteur, qui est lui-même de préférence organique.

Ce matériau est biocompatible, bioactif, biorésorbable et comme on le voit sur les figures 1 et 2, il a une morphologie très régulière, en termes de distribution de pores, et en termes de forme de pores.

De préférence, ce matériau a des pores, en forme de sphères dont le diamètre, soit est identique en tous points, soit en forme de sphères dont le rapport du plus petit diamètre au plus grand diamètre est de 0,9 ± 0,1, au maximum, soit en forme de polyèdres s'inscrivant dans une telle sphère, les différences entre les diamètres en différents points du polyèdre s'inscrivant dans cette sphère étant d'au plus ± 15 % du diamètre de la sphère dans laquelle ils s'inscrivent.

Les matériaux d'implant de l'invention peuvent avoir des tailles de pores se situant dans un très large intervalle de 100 à 900 µm, de préférence 200 µm à 800 µm, bornes incluses, avec un écart entre le diamètre de la sphère la plus petite ou la plus grande étant au plus de 70%, de préférence au plus de 50%, plus préférablement au plus de 30%, par rapport au diamètre moyen arithmétique de l'ensemble des sphères de l'implant en association avec des interconnexions entre pores dont la plus petite dimension est comprise entre 25 µm et 250 µm, bornes incluses.

Au moins 70% en nombre de ces pores a au moins une interconnexion avec un autre pore.

Une telle forme et distribution de tailles de pores ainsi que de telles tailles d'interconnexions entre pores sont très favorables à la conduction des cellules, à la repousse osseuse et à l'invasion tissulaire comme cela a été démontré dans la demande de brevet EP 3003414.

La matrice est constituée d'une phase organique, d'une phase inorganique et d'un nutriment ostéoinducteur pour augmenter le potentiel ostéoinducteur du matériau d'implant de l'art antérieur.

La phase inorganique est le verre bioactif M.

Les céramiques bioactives et les verres bioactifs sont bien connus de l'homme du métier et sont décrits en particulier dans L.L. Hench et al., J. Biomed. Mater. Res. 1971, 2, 117-141 ; L.L. Hench et al., J. Biomed. Mater. Res. 1973, 7, 25-42 pour les céramiques bioactives et dans M. Vallet-Regi et al., Eur. J. Inorg. Chem. 2003, 1029-1042 et WO 02/04606, WO 00/76486 et WO 2009/027594, en particulier. Dans l'invention, on utilise uniquement un verre bioactif.

La partie organique du matériau d'implant de l'invention comprend le polymère biodégradable P et le nutriment ostéoinducteur N. De préférence, le nutriment ostéoinducteur est une molécule organique.

Le polymère biodégradable P est soluble dans au moins un solvant S1 et insoluble dans au moins un solvant S. Ces solvants peuvent être de l'eau, un milieu aqueux ou encore un solvant organique.

De préférence, le polymère biodégradable P est choisi parmi :
- les polysaccharides biorésorbables, de préférence choisis parmi le dextran, l'acide hyaluronique, l'agar, le chitosane, l'acide alginique, l'alginate de sodium ou de potassium, le galactomannane, le carraghénane, la pectine,
- les polyesters biorésorbables, de préférence l'alcool polyvinylique ou le poly(acide lactique), et
- les polymères synthétiques biodégradables, de préférence un polyéthylène glycol, ou le poly(caprolactone).

Le nutriment ostéoinducteur N est soluble dans au moins un solvant S2 miscible avec le solvant S1, et insoluble dans le solvant S.

Le solvant S2 ne doit pas dégrader les propriétés du matériau d'implant final : il ne doit pas entraîner une séparation des phases organique et inorganique, ni modifier l'homogénéité de ces phases dans le matériau final. Il ne doit pas non plus entraîner une perte des propriétés mécaniques du matériau d'implant final : celui-ci doit rester manipulable, par exemple pour pouvoir être éventuellement mis à la forme et à la taille voulues pour l'implant final, pour que l'implant fabriqué avec ce matériau puisse être manipulé par le chirurgien qui va l'implanter.

De préférence, le nutriment ostéoinducteur N est choisi parmi la vitamine D2 (ergocalciférol), la vitamine D3 (cholicalciférol), leurs dérivés et précurseurs, la vitamine K1, la vitamine K2, les acides gras oméga-3, l'acide punicique, l'acide α-lipoïque, les anthocyanines, les flavonols, les procyanidines, le tyrosol, l'oleuropéïne, la naringénine, la punicalagine, l'acide ellagique et la phycocyanine.

La matrice du matériau d'implant de l'invention est constituée du verre bioactif M et du polymère biodégradable P, qui forment un matériau hybride, c'est-à-dire formant une seule phase et dans laquelle est incorporé le nutriment ostéoinducteur N.

Dans un premier mode de réalisation préféré, dans le matériau d'implant de l'invention, le matériau hybride dopé en un nutriment ostéinducteur comprend 30% en masse de verre bioactif M à base de SiO₂ et CaO, par rapport à la masse totale (verre bioactif M + polymère biodégradable P + nutriment ostéoinducteur N), 69% en masse de poly(caprolactone), par rapport à la masse totale (verre bioactif M + polymère biodégradable P + nutriment ostéoinducteur N), et 1% en masse de fisétine et/ou d'hydroxytyrosol, par rapport à la masse totale (verre bioactif M + polymère biodégradable P + nutriment ostéoinducteur N).

Dans un second mode de réalisation préféré, dans le matériau d'implant de l'invention, le matériau hybride dopé en un nutriment ostéinducteur comprend 40% en masse de verre bioactif M à base de SiO₂ et CaO, par rapport à la masse totale (verre bioactif M + polymère biodégradable P + nutriment ostéoinducteur N), 59% en masse de poly(caprolactone), par rapport à la masse totale (verre bioactif M + polymère biodégradable P + nutriment ostéoinducteur N), et 1% en masse de fisétine et/ou d'hydroxytyrosol, par rapport à la masse totale (verre bioactif M + polymère biodégradable P + nutriment ostéoinducteur N).

Le matériau hybride utilisé dans l'invention est obtenu par un procédé qui est aussi un objet de l'invention.

Ce procédé comprend la formation d'un sol contenant tous les précurseurs alcoxydes du verre bioactif, la solubilisation du polymère biodégradable P dans le solvant S1, la solubilisation du nutriment ostéoinducteur N dans le solvant S2, l'ajout de la solution de nutriment ostéoinducteur N dans la solution du polymère biodégradable P et leur mélange jusqu'à obtenir un mélange uniforme, c'est-à-dire sans séparation de phases, l'ajout de ce mélange polymère biodégradable P- nutriment ostéoinducteur N dans le sol contenant tous les précurseurs alcoxydes du verre bioactif et la gélification de la solution ainsi obtenue par une succession de réactions de polymérisation (polymérisation sol-gel de la phase inorganique) (condensation des alcoxydes). On obtient alors un mélange hybride associant intimement la phase minérale et la phase organique.

Le matériau hybride se distingue donc du matériau composite par une intrication intime entre les deux phases organique et inorganique, ces deux phases étant indiscernables (sauf à l'échelle moléculaire) dans le cas d'un mélange hybride.

Dans un mode de réalisation préféré, le matériau d'implant de l'invention est obtenu par un procédé faisant appel à un agent porogène A qui est constitué de microsphères en un polymère soluble dans au moins un solvant S dans lequel le polymère biodégradable P et le nutriment ostéoinducteur N ne sont pas solubles.

Pour ne pas dégrader les propriétés mécaniques et morphologiques du matériau d'implant de l'invention, le solvant S2 utilisé pour solubiliser le nutriment ostéoinducteur N ne doit pas solubiliser l'agent porogène A ou le dégrader. Il ne doit pas non plus dégrader le polymère biodégradable P ou le verre bioactif M. De plus, il doit être miscible avec le solvant S1. De préférence, le solvant S2 est identique au solvant S1.

Ensuite, le procédé de l'invention consiste à empiler des microsphères d'agent porogène A en un matériau polymère, différent du polymère biodégradable P, dans un moule ayant la forme et la taille correspondant à la géométrie du défaut osseux à combler ou du défaut où la régénération osseuse est voulue.

Ces microsphères d'agent porogène A permettent d'obtenir au final des pores dont la taille et la distribution correspondront en négatif à l'empilement de microsphères.

En effet, le matériau destiné à constituer la matrice sera ensuite infiltré dans l'empilement des billes de microsphères d'agents porogènes A, puis solidifié pour pouvoir être démoulé sans changer la forme et la taille de l'empilement de l'implant voulu. L'agent porogène A sera alors éliminé permettant l'obtention du matériau d'implant de l'invention.

Comme on le voit, ce procédé n'utilise aucun traitement thermique à haute température pour fritter le verre bioactif M, la seule température nécessaire étant la température d'évaporation du solvant S utilisé.

Comme cela apparaîtra clairement, l'invention réside sur la judicieuse association du choix de différents matériaux et de différents solvants :
1) le matériau constituant le polymère biodégradable P,
2) le matériau constituant l'agent porogène A,
3) le nutriment ostéoinducteur N,
4) le solvant S de l'agent porogène A qui ne doit ni dissoudre ni dégrader le polymère biodégradable P ,le nutriment ostéoinducteur N et le verre bioactif M,
5) les solvants S1 et S2 qui peuvent être identiques ou différents mais qui doivent être miscibles ensemble, ne pas dissoudre l'agent porogène A et ne dégrader ni le verre bioactif M, ni le polymère biodégradable P, ni le nutriment ostéoinducteur N.

On comprend alors que le choix des différents matériaux et solvants ne peut se faire indépendamment du choix des autres.

Parmi les polymères biodégradables P préférés utilisables se trouvent :
- les polysaccharides biorésorbables, de préférence choisis parmi le dextran, l'acide hyaluronique, l'agar, le chitosane, l'acide alginique, l'alginate de sodium ou de potassium, le galactomannane, le carraghénane, la pectine ;
- les polyesters biorésorbables, de préférence l'alcool polyvinylique; ou l'acide polylactique, et
- les polymères synthétiques biodégradables, de préférence un polyéthylène glycol, ou le poly(caprolactone).

Alors que dans la demande de brevet EP3003414, les protéines sont citées comme polymère biodégradable utilisable, dans l'invention les protéines ne font pas partie des polymères biodégradables P utilisable, en raison de la difficulté à les solubiliser en présence des nutriments ostéoinducteurs proposés ci-avant : elles sont insolubles ou pratiquement insolubles dans de nombreux solvants organiques tels qu'un alcool, le tétrahydrofurane, etc. et elles sont peu solubles dans l'eau.

Des exemples de matériaux pour l'agent porogène A sont les polymères biodégradables insolubles dans un milieu aqueux et solubles dans le au moins un solvant S, de préférence choisi parmi les polyméthacrylates d'alkyle en C₁ à C₄, de préférence le polyméthacrylate de méthyle ou le polyméthacrylate de butyle, le polyuréthane, l'acide polyglycolique, les différentes formes d'acides polylactiques, les copolymères d'acides lactique-coglycoliques, le poly(caprolactone), le polypropylène fumarate, la paraffine et le naphtalène, ou l'acrylonitrile butadiène styrène (ABS).

Les solvants S sont en particulier l'acétone, l'éthanol, le chloroforme, le dichlorométhane, l'hexane, le cyclohexane, le benzène, l'éther diéthylique, l'hexafluoroisopropanol, et le tétrahydrofurane (THF).

Des exemples de nutriments ostéoinducteurs N sont la vitamine D (vitamine D2 (ergocalciférol) et vitamine D3 (cholicalciférol), ses dérivés et précurseurs, la vitamine K1, la vitamine K2, les acides gras oméga-3, l'acide punicique, l'acide α-lipoïque, les anthocyanines, les flavonols, les procyanidines, le tyrosol, l'oleuropéïne, la naringénine, la punicalagine, l'acide ellagique et la phycocyanine.

Le collagène hydrolysé ne fait pas partie des nutriments ostéoinducteurs utilisables dans l'invention. Comme on le verra à l'exemple 5 ci-après, le matériau d'implant en un matériau hybride poly(caprolactone)-verre bioactif- collagène hydrolysé n'a pas une tenue mécanique suffisante, en raison de la nécessité d'employer un solvant THF-acide acétique pour solubiliser le mélange PCL - collagène hydrolysé.

Dans l'invention, de préférence, le polymère biodégradable P est le poly(caprolactone) (PCL), les microsphères d'agent porogène A sont en paraffine, le solvant S est le cyclohexane, le nutriment ostéo inducteur N est la fisétine et/ou l'hydroxytyrosol, le solvant S1 est identique au solvant S2 et est le tétrahydrofurane (THF).

Dans le procédé de fabrication du matériau d'implant hybride de l'invention, l'introduction des microsphères dans le moule peut être faite avant l'introduction du mélange des précurseurs alcoxydes du verre bioactif M, du polymère biodégradable P et du nutriment ostéoinducteur N.

Cependant, il est également possible de d'abord introduire le mélange des précurseurs alcoxydes du verre bioactif M, du polymère biodégradable P et du nutriment ostéoinducteur N dans le moule, et d'y verser ensuite les microsphères d'agent porogène A.

Il est encore possible de réaliser un mélange des précurseurs alcoxydes du verre bioactif M, du polymère biodégradable P, du nutriment ostéoinducteur N et des microsphères d'agent porogène A et d'introduire le tout dans le moule.

Pour obtenir un matériau dont au moins 70% en nombre de pores, ont au moins une interconnexion avec un autre pore, la quantité d'agent porogène A introduite dans le mélange polymère biodégradable P-verre bioactif M doit représenter au moins 60% en volume du volume total du mélange polymère biodégradable P-verre bioactif M-nutriment ostéoinducteur N - agent porogène A introduit dans le moule.

La taille des interconnexions est liée à la taille du point de contact entre les sphères d'agent porogène A dans l'empilement de sphères réalisé. L'augmentation de la taille des interconnexions générées, à diamètre poreux constant, est possible moyennant l'ajout d'une étape consistant en une fusion partielle des sphères porogènes dans l'empilement initialement réalisé, de manière à augmenter la taille de leur point de contact.

Les microsphères d'agent porogène A doivent former un empilement compact, lorsque placées dans le moule avec, dans l'invention, le sol des précurseurs alcoxydes du verre bioactif M, le polymère biodégradable P et le nutriment ostéoinducteur N.

Pour cela, le volume d'agent porogène A, par rapport au volume total du mélange polymère biodégradable P-précurseurs du verre bioactif M-agent porogène A-nutriment ostéoinducteur N, doit être d'au moins 60%, de préférence d'au moins 70%.

Quant au rapport en masse biopolymère P/verre bioactif M, il pourra être compris entre 10/90 et 90/10. De préférence, pour des raisons de tenue mécanique du matériau obtenu, il sera compris entre 20/80 et 80/20, la meilleure tenue mécanique (manipulation aisée sans déformations ou perte de matière) étant obtenue avec un rapport 70/30.

La quantité de nutriment ostéoinducteur N peut varier entre 0,1% et 10%, de préférence entre 0,1% et 5%, en masse par rapport à la masse totale (verre bioactif M + polymère biodégradable P + nutriment ostéoinducteur N).

Dans le cas de la fisétine et de l'hydroxytyrosol, une quantité de 1% en masse par rapport à la masse totale (verre bioactif M + polymère biodégradable P + nutriment ostéoinducteur N) est suffisante.

### Exemples

Afin de mieux faire comprendre l'invention, nous allons en décrire maintenant, à titre purement illustratif et non limitatif, plusieurs exemples de mise en oeuvre.

### Exemple 1 : (comparatif) Fabrication d'un matériau d'implant selon l'art antérieur à matrice en matériau hybride non dopé dans lequel le polymère biodégradable P est le poly(caprolactone) et le verre bioactif M est constitué de 75% de SiO₂ et 25% de CaO, en masse, par rapport à la masse totale du verre.

La première étape était l'empilement des microsphères d'agent porogène A en paraffine dans un moule ayant la géométrie recherchée pour l'implant.

Le volume des microsphères d'agent porogène A représentait 70% du volume total du mélange agent porogène A-polymère biodégradable P-précurseurs du verre bioactif M.

L'agent porogène était sous la forme de particules sphériques, à savoir d'un diamètre compris entre 400 et 600µm.

Leurs diamètres peuvent être choisis entre plusieurs dizaines à plusieurs centaines de microns, suivant les applications. La porosité du matériau d'implant de l'invention qui sera finalement obtenu peut être contrôlée sur ces deux points ; premièrement le diamètre des pores qui sera obtenu dépend directement du diamètre des particules porogènes initiales. Il suffit donc d'ajuster la granulométrie des microsphères de paraffine initiales en vue d'obtenir très simplement la porosité désirée. Deuxièmement la taille des interconnexions entre pores dépend directement de la taille de la zone de contact entre les billes polymères dans l'empilement initial. La taille de cette zone de contact peut être modifiée en faisant fusionner entre elles les particules de polymère initiales, au moyen d'un solvant S, ou par un traitement thermique préliminaire. Cette procédure a déjà été décrite par Descamps et al., "Manufacture of macroporous beta-tricalcium phosphate bioceramics". Journal of the European Ceramic Society 2008, 28, (1), 149-157 et "Synthesis of macroporous beta-tricalcium phosphate with controlled porous architectural". Ceramics International 2008, 34, (5), 1131-1137.

A la deuxième étape, le poly(caprolactone) a été mis en solution dans du tétrahydrofurane (THF).

Dans une troisième étape, la solution de poly(caprolactone) a été versée dans le sol contenant les précurseurs alcoxydes du verre bioactif.

Les précurseurs alcoxydes du verre bioactif étaient les suivants
- Tétraéthylorthosilicate TEOS
- Ethoxyde de Calcium Ca(OEt)₂
- HCl 2M.

Ils étaient en des quantités telles que la composition du verre bioactif était 75% SiO₂ et 25% CaO, en masse, par rapport à la masse total du verre bioactif obtenu au final.

Dans une quatrième étape, le mélange hybride ci-dessus, a été versé dans le moule contenant l'empilement de microsphères de paraffine.

Une centrifugation ou une infiltration sous pression ou une infiltration sous vide peuvent être utilisées pour aider le mélange hybride à remplir les interstices entre les microsphères de paraffine.

Le matériau hybride a été obtenu par un procédé sol-gel.

Dans ce procédé, un sol contenant tous les précurseurs alcoxydes du verre bioactif et le polymère biodégradable en solution est amené à gélifier par une succession de réactions de polymérisation.

Cette gélification est menée à une température comprise entre 0°C et 60°C, inclus, afin de ne pas dégrader la matrice en matériau hybride obtenue.

Une fois solidifié, le matériau d'implant contenant les microsphères de paraffine est démoulé, et les microsphères d'agent porogène en paraffine sont éliminées par lavage avec du cyclohexane.

Après plusieurs étapes de lavage, l'empreinte initiale des microsphères de paraffine est complètement éliminée et le matériau final est obtenu, sous la forme d'un bloc macroporeux hybride en verre bioactif/poly(caprolactone).

On a obtenu un matériau d'implant constitué de 70% en masse de poly(caprolactone) et de 30% en masse de verre bioactif et ayant au moins 70%, en nombre de pores ayant au moins une interconnexion avec un autre pore.

La structure obtenue peut être lavée sans aucun dommage dans des bains d'éthanol, afin d'éliminer d'éventuels résidus indésirables, tels que des chlorures, du THF, etc.

### Exemple 2 : (selon l'invention) Fabrication d'un matériau d'implant à matrice en matériau hybride dopé en nutriment ostéoinducteur dans lequel le polymère biodégradable P est le poly(caprolactone), le verre bioactif M est constitué de 75% de SiO₂ et 25% de CaO, en masse, par rapport à la masse totale du verre et le nutriment ostéoinducteur N est la fisétine.

La première étape était l'empilement des microsphères d'agent porogène A en paraffine dans un moule ayant la géométrie recherchée pour l'implant.

Le volume des microsphères d'agent porogène A représentait 70% du volume total du mélange agent porogène A-polymère biodégradable P-nutriment ostéoinducteur N-précurseurs du verre bioactif M, comme à l'exemple 1.

L'agent porogène était sous la forme de particules sphériques, à savoir d'un diamètre compris entre 400 et 600 µm, comme à l'exemple 1.

A la deuxième étape, le poly(caprolactone) a été mis en solution dans du tétrahydrofurane (THF), comme à l'exemple 1.

Séparément, la fisétine a été solubilisée dans la solution de poly(caprolactone) ainsi obtenue. Cela est possible car la fisétine est soluble dans le THF. Le mélange est agité jusqu'à obtention d'une solution homogène, c'est-à-dire sans séparation de phases.

La quantité de fisétine introduite était telle que sa masse dans le matériau d'implant final représentait 1% de la masse (verre bioactif plus poly(caprolactone) plus fisétine).

La solution obtenue a une teinte rouge en raison de la présence de la fisétine.

Dans une troisième étape, la solution de poly(caprolactone) - fisétine a été versée dans le sol contenant les précurseurs alcoxydes du verre bioactif.

Les précurseurs alcoxydes du verre bioactif étaient les mêmes que ceux utilisés à l'exemple 1 et dans les mêmes quantités qu'à l'exemple 1.

Dans une quatrième étape, le mélange hybride ci-dessus, a été versé dans le moule contenant l'empilement de microsphères de paraffine.

Une centrifugation ou une infiltration sous pression ou une infiltration sous vide peuvent être utilisées pour aider le mélange hybride à remplir les interstices entre les microsphères de paraffine.

Une gélification est menée à une température comprise entre 0°C et 60°C, inclus, afin de ne pas dégrader la matrice en matériau hybride obtenue.

Une fois solidifié, le matériau d'implant contenant les microsphères de paraffine est démoulé, et les microsphères d'agent porogène en paraffine sont éliminées par lavage avec du cyclohexane.

Après plusieurs étapes de lavage, l'empreinte initiale des microsphères de paraffine est complètement éliminée et le matériau final est obtenu, sous la forme d'un bloc macroporeux hybride en verre bioactif/poly(caprolactone)/fisétine.

On a obtenu un matériau d'implant, de couleur rougeâtre, constitué de 69% en masse de poly(caprolactone) , de 30% en masse de verre bioactif et de 1% en masse de fisétine, et ayant 70%, en nombre de pores ayant au moins une interconnexion avec un autre pore.

La structure obtenue peut être lavée sans aucun dommage dans des bains d'éthanol, afin d'éliminer d'éventuels résidus indésirables, tels que des chlorures, du THF, etc.

Lors des lavages avec le cyclohexane pour éliminer les microsphères de paraffine et avec l'éthanol, les solvants restent incolores, ce qui indique un relargage négligeable, voire inexistant de la fisétine.

### Exemple 3 : (selon l'invention) Fabrication d'un matériau d'implant à matrice en matériau hybride dopé en nutriment ostéoinducteur dans lequel le polymère biodégradable P est le poly(caprolactone), le verre bioactif M est constitué de 75% de SiO₂ et 25% de CaO, en masse, par rapport à la masse totale du verre et le nutriment ostéoinducteur N est l'hydroxytyrosol.

Le procédé est identique à l'exemple 2 mais en remplaçant la fisétine par l'hydroxytyrosol.

La solution obtenue lors de la solubilisation de l'hydroxytyrosol dans la solution de poly(caprolactone), ainsi que le matériau d'implant obtenu au final a une teinte jaune en raison de la présence de l'hydroxytyrosol.

A cet exemple également, lors des lavages avec le cyclohexane pour éliminer les microsphères de paraffine et avec l'éthanol, les solvants restent incolores, ce qui indique un relargage négligeable, voire inexistant de l'hydroxytyrosol.

### Exemple 4 : (comparatif) Fabrication d'un matériau d'implant à matrice en un matériau hybride dans lequel le polymère biodégradable P est le collagène hydrolysé et le verre bioactif M est constitué de 75% de SiO₂ et 25% de CaO, en masse, par rapport à la masse totale du verre.

Le collagène hydrolysé ne gonfle pas au contact des fluides physiologiques.

De plus, tout comme la gélatine et le collagène, le collagène hydrolysé possède des séquences d'acides aminés qui servent de récepteur aux intégrines et favorisent de ce fait l'adhésion cellulaire.

Le collagène hydrolysé est produit à partir du collagène. Le collagène est une protéine composée de trois chaînes polypeptidiques, reliées entre elles par des liaisons hydrogènes et des liaisons covalentes. Il est généralement extrait de la peau de porc ou de boeuf. L'hydrolyse partielle du collagène brise les liaisons intermoléculaires et mène à la dissociation des brins ; la gélatine est alors obtenue. L'hydrolyse avancée de la gélatine conduit ensuite à la rupture de liaisons peptidiques, et les brins initialement constitués de plus d'un millier d'acides aminés sont décomposés en peptides d'une vingtaine d'acides aminés ; le collagène hydrolysé est ainsi obtenu.

Le collagène hydrolysé est dissout dans l'eau, puis mélangé au sol de bioverre. Le sol hybride résultant contient des particules blanches qui lui donnent une couleur blanche et un aspect opaque. Après homogénéisation par agitation et ultrasons, le sol hybride est versé sur un empilement de billes en paraffine et le tout est ensuite centrifugé à 3900 tr/min pendant 3 min. Cette centrifugation ne permet pas l'infiltration du sol hybride collagène hydrolysé-bioverre. En effet, on observe un dépôt blanc sur l'empilement de billes, au-dessus duquel surnage une solution limpide.

Au vu de cette impossibilité d'obtenir un mélange homogène collagène hydrolysé-bioverre, des études ont été menées et ont alors montré que le collagène hydrolysé et le bioverre sont tous deux solubles dans des mélanges acide acétique (acide organique faible)-eau contenant 90% d'acide acétique. Les analyses en chromatographie - spectrométrie de masse démontrent que l'acide acétique ne dégrade pas le collagène hydrolysé.

Le collagène hydrolysé est alors dissout à une concentration de 0,30 g/mL dans un mélange acide acétique-eau contenant 90 % volumique d'acide acétique. Puis le sol de bioverre est ajouté de sorte à atteindre une composition massique de 70 % polymère 30 % bioverre.

La solution hybride obtenue est limpide. Elle est ensuite coulée sur l'empilement de billes et centrifugée.

Une gélification est menée à une température comprise entre 0°C et 60°C, inclus, afin de ne pas dégrader la matrice en matériau hybride obtenue.

Une fois solidifié, le matériau d'implant contenant les microsphères de paraffine est démoulé, et les microsphères d'agent porogène en paraffine sont éliminées par lavage avec du cyclohexane.

Le matériau d'implant fabriqué possède des propriétés mécaniques faibles et se dissout rapidement dans l'eau. Il est effectivement nécessaire de le manipuler avec délicatesse, sans quoi il tend à se briser.
Il en résulte que le dopage du bioverre présenté ici avec le collagène hydrolysé n'est pas adapté au comblement osseux.

### Exemple 5 : (comparatif) : Fabrication d'un matériau d'implant à matrice en un matériau hybride dopé en nutriment ostéoinducteur dans lequel le polymère biodégradable P est le poly(caprolactone), le verre bioactif M est constitué de 75% de SiO₂ et 25% de CaO, en masse, par rapport à la masse totale du verre et le nutriment ostéoinducteur N est le collagène hydrolysé.

Comme déjà dit à l'exemple 4, le collagène hydrolysé stimule l'activité des ostéoblastes, améliore l'absorption du calcium et possède des propriétés anti-inflammatoires et anti-oxydantes.

Bien que la synthèse de matériau d'implant d'hybride collagène hydrolysé-bioverre ait été abandonnée, il est apparu intéressant d'incorporer cette protéine en faible proportion dans la trame organique de l'hybride PCL-bioverre, un dopage organique en collagène hydrolysé pourrait permettre d'améliorer le potentiel ostéoinducteur de l'hybride. Le collagène hydrolysé est substitué au PCL. On vise une composition massique de 60 % PCL, 10 % collagène hydrolysé et 30 % bioverre SiO2-CaO que l'on note PCL-colH.

Les études précédentes ont permis de révéler l'acide acétique comme solvant commun du PCL et du collagène hydrolysé (cf. exemple 4). L'acide acétique permet la dissolution des constituants organiques de façon homogène et la réalisation du sol hybride, mais après dissolution des billes de paraffine (porogène) les matériaux d'implants se désintègrent.

Des matériaux d'implants sont alors fabriqués en utilisant comme solvant commun du poly(caprolactone) et du collagène hydrolysé des mélanges de composition volumique 80 % acide acétique-20 % THF. Ils ne se désintègrent pas et possèdent des pores et des interconnexions bien définis et dont les diamètres sont identiques à ceux des scaffolds hybrides PCL-bioverre non-dopés.

Cependant, la découpe des matériaux d'implant obtenus n'est pas nette et s'accompagne d'un léger déchirement des murs.

Le relargage du collagène hydrolysé est étudié à travers une interaction semi-dynamique dans le SBF (Simulated Body Fluid) à raison de 1 mL par mg de matériau. Le SBF est renouvelé tous les 2 jours et le SBF interagi est analysé avec un kit de BioAssay afin de déterminer la concentration en collagène hydrolysé. L'étude compte 6 interactions consécutives de 2 jours. Les analyses révèlent un relargage de 40 % du collagène hydrolysé dans le SBF après la première interaction, puis la concentration en collagène hydrolysé dans le SBF est en-dessous du seuil de détection pour les interactions suivantes. Ces résultats démontrent un relargage rapide du collagène hydrolysé. Le relargage rapide était attendu puisque le collagène hydrolysé est soluble dans l'eau et est simplement intriqué aux chaînes de PCL (pas de liaison covalente). Ainsi, on peut conclure que la potentielle action de ce dopage organique se fera sur le court terme.

Le potentiel ostéoinducteur du matériau d'implant PCL-colH est évalué *in vitro* et comparé à celui de l'hybride non-dopé (PCL-BV) et de l'os trabéculaire bovin (OTB). Les matériaux d'implant sont ensemencés avec des ostéoblastes primaires de rats et l'activité de l'ALP (phosphatase alcaline) est déterminée après 7 jours et 14 jours de culture cellulaire. L'activité ALP est significativement plus importante avec le PCL-colH après 7 jours, puis elle est similaire pour le PCL-colH et le PCL-BV après 14 jours. Le collagène hydrolysé stimule donc le processus de minéralisation, mais cet effet se limite à des temps courts comme attendu. Sachant que le SBF est renouvelé tous les 2 à 3 jours dans cette expérience, il se peut qu'après 7 jours de culture cellulaire (c'est-à-dire 1 pré-incubation et 3 interactions) il ne subsiste plus de collagène hydrolysé dans l'hybride qui n'en relargue alors plus dans le milieu, ce qui expliquerait une ostéoinduction limitée aux temps précoces. Ainsi, ces résultats cellulaires rejoignent les observations précédentes sur le relargage rapide du collagène hydrolysé.

Les matériaux d'implant PCL-colH sont finalement implantés dans la calvaria de souris. Après 3 mois d'implantation, l'os ne s'est pas régénéré dans les matériaux d'implants PCL-colH alors que la repousse osseuse est bien avancée dans les matériaux d'implants non-dopés. Cette observation est corrélée à une activité des ostéoblastes faible dans la zone d'implantation du PCL-colH illustrée par l'absence d'ALP.

De plus, on constate une inflammation importante avec le PCL-colH.

De ce fait, le matériau PCL-colH ne semble pas adapté au comblement osseux, contrairement à l'hybride PCL-BV même non-dopé comme en témoingnent les performances *in vivo.*

### Exemple 6 : Evaluation des propriétés mécaniques des matériaux d'implant obtenus aux exemples 1 à 3.

La manipulation des matériaux d'implant obtenus aux exemples 1 à 3 permet de constater que les matériaux d'implant dopés obtenus aux exemples 2 et 3 ont des propriétés mécaniques identiques à celles du matériau d'implant de l'art antérieur obtenu à l'exemple 1 : ils ont une bonne tenue mécanique, ils sont à la fois légèrement élastiques et suffisamment rigides pour permettre leur manipulation. Leur découpe au scalpel est simple et propre. Du point de vue macroscopique il n'est pas possible de différencier les matériaux dopés du matériau non dopé, hormis par la couleur.
L'examen au microscope électronique à balayage ne montre pas de différence de morphologie et d'état de surface.
Comme illustré en figures 1 et 2, le matériau d'implant non dopé a une structure identique à celle des matériaux d'implant dopés, avec des pores bien définis et fortement interconnectés, une rugosité micrométrique en surface des parois et une porosité micrométrique à l'intérieur des murs.

### Exemple 7 : Evaluation in vitro des implants obtenus aux exemples 1 à 3.

Les potentiels ostéoinducteurs des matériaux d'implant hybrides dopés en fisétine (PCL-fis) (exemple 2) ou en hydroxytyrosol (PCL-hyd) (exemple 3) sont évalués *in vitro* et comparés à celui de l'hybride non-dopé (PCL-BV) (exemple 1).

Les matériaux d'implant sont ensemencés avec des ostéoblastes primaires de rats et l'activité de l'ALP est déterminée après 7 jours et 14 jours de culture cellulaire. L'activité ALP est normalisée avec la quantité relative GAPDH présente dans chaque échantillon . La culture est réalisée sous agitation orbitale (10 rpm).

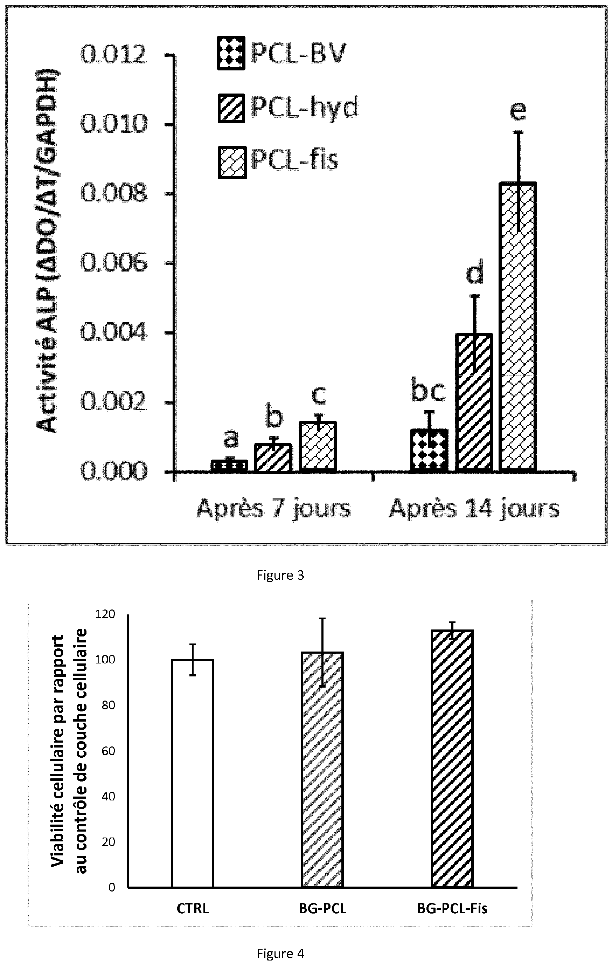

Comme on le voit en figure 3 qui montre les résultats quantitatifs sur la différenciation cellulaire au contact de ces matériaux, sous la forme d'histogrammes ( traitement statistique ANOVA suivi de Tukey pour un intervalle de confiance de 95 %), des différences significatives sont observées entre les trois matériaux aux deux temps. L'activité ALP est plus importante avec PCL-fis, puis PCL-hyd et enfin PCL-BV, démontrant ainsi un effet ostéoinducteur de ces dopants organiques.

L'effet ostéoinducteur de PCL-fis et PCL-hyd est observé jusqu'à 14 jours de culture cellulaire, qui équivalent à 1 pré-incubation et 6 interactions. Il semble donc que le relargage de ces dopants se fasse de façon durable. On observe d'ailleurs un changement de couleur des milieux de culture après interaction avec PCL-fis, ceux-ci prenant la teinte du dopant.

Ce changement de couleur s'atténue au fil des renouvellements du milieu mais il est toujours notable, ce qui indique un relargage étalé dans le temps. Le relargage progressif de la fisétine et de l'hydroxytyrosol est à relier à une faible solubilité des composés phénoliques en milieu aqueux et de l'hybride à base de PCL.

En conclusion, la fisétine et l'hydroxytyrosol améliorent le potentiel ostéoinducteur de l'hybride. Du point de vue économique également, l'utilisation de ces antioxydants en tant que constituants d'un biomatériau représente une stratégie bien plus intéressante comparée à l'ingestion de compléments alimentaires post-opératoires. De nombreux composés organiques ostéoinducteurs peuvent également être envisagés pour le dopage organique de l'hybride PCL-bioverre, en particulier dans la famille des polyphénols (oleuropéine, hespéridine, naringine, resveratrol).

### Exemple 8 : Evaluation in vivo des implants obtenus aux exemples 1 et 2.

### Respect de l'éthique et de l'animal

Toutes les expériences ont été effectuées en suivant un protocole approuvé par le Comité du bien être des animaux de l'Université de Paris Descartes (agrément de projet 17-093, APAFIS N°2018031514511875). Les animaux ont été traités selon les conditions éthiques développées par les directives du conseil de l'Union Européenne (accord sur élevage d'animaux C92-049-01). Tous les efforts ont été faits pour minimiser leur douleur ou inconfort. Les souris C57b16 ont été achetées à Janvier Labs (Le Genest Saint Isle, France). Elles ont été logées à 22 ± 2°C avec un cycle jour/nuit de 12h, et un accès *ad libitum* à l'eau et la nourriture dans le bâtiment du Département des pathologies, imagerie et biothérapies Orofaciales de l'Université Descartes, Montrouge, France.

### Implantation chirurgicale, échantillonnage et procédure expérimentale

Les souris C57b16 (âgées de 12 semaines, environ 30g) ont été anesthésiées par injection intrapéritonéale de kétamine (80 mg/kg) et de xylazine (10 mg/kg), provenant toutes deux de Centravet Alfort, Maisons Alfort, France.

La peau du cuir chevelu a été incisée et le périosteum a été écarté pour visualiser le crâne. Un défaut de taille critique de 3,5 mm de diamètre de la calotte crânienne a été crée de chaque côté de l'os pariétal un utilisant un poinçon Tissue ^{®} (provenant de Praxis l'Instrumentiste, France) lié à une pièce à main à vitesse lente fonctionnant à 1500rpm, sous irrigation de solution saline stérile.

Un soin spécial a été pour la préservation de la suture sagittale, et une invasion minimale de la dure mère.

Après avoir doucement éliminé le bouchon d'os, les défauts ont été remplis avec l'implant constitué du matériau hybride dopé à la fisétine (BG-PCL-Fis) obtenu à l'exemple 2 ou l'implant non dopé de l'exemple 1 (BG-PCL).

Les dimensions des implants cylindriques avaient un diamètre de 3,5 mm et une hauteur de 1 mm (n=8).

Pour chaque animal, le même type de matériau a été implanté dans les deux défauts.

Les défauts créés dans les crânes de six souris additionnelles ont été laissés vides en tant que contrôles négatifs (chirurgie « Sham ») pour contrôler le caractère critique du défaut. La fermeture des incisions est terminée par une suture absorbables (Vicryl Rapid ^{®} 4.0, Ethicon, Johnson & Johnson). Un soin post-opératoire immédiat a été appliqué par analgésie avec de la buprénorphine (0,02 mg/kg de poids corporel). Après chirurgie, les animaux ont été logés individuellement sous observation constante.

Aucune létalité n'a été constatée pendant la chirurgie et la période post-opératoire.

La cicatrisation a progressé sans aucun signe d'infection, d'exposition de matériel ou autre complications.

Les masses corporels ont été surveillées régulièrement pour s'assurer d'une alimentation appropriée avant et après la chirurgie.

Aux jours 0, 30, 60 et 90 post-chirurgie, le crâne des animaux ont été visualisés par micro--tomographie aux rayons X (micro-CT) comme décrit ci-après.

Tous les animaux ont été euthanasiés au jour 90 et leurs calottes crâniennes ont été excisées.

Les échantillons ont été fixés dans de l'éthanol à 70% v/v (24h à 4°C), puis déshydratés dans des solutions d'éthanol comprenant des %, en volume, croissants d'éthanol pour éliminer toute trace d'eau, et enrobés à - 20°C dans une résine de méthyl méthacrylate (Merck) sans décalcification.

Les échantillons d'os de la calotte crânienne enrobés de résine ont été coupés (5 mm d'épaisseur) en utilisant un microtome Jung Polycut E^{®} (Leica) avec des lames pour tissus durs (Leica).

Après immersion dans une goutte d'éthanol à 50% v/v, les sections ont été étirées à un état sans plis sur des lames de verre revêtues de gélatine (Menzel-Gläser), revêtues d'une feuille de polyéthylène, et pressées étroitement sur les lames de verre, et laissées à sécher une nuit à température ambiante.

Une déplastification a été mise en oeuvre dans du 2-méthoxyéthyl acétate (Carlo Erba) trois fois pendant 20miutes.

Une réhydratation des sections a été effectuée à l'aide de solution d'éthanol de concentrations décroissantes pour les procédures suivantes.

### Micro-Tomographie par rayon X

Les souris ont été anesthésiées (isoflurane, induction à 3-4% sous flux d'air de 0,8 à 1,5 L/min ; 1,5 à 2% sous 400 à 800mL/min après) à la ligne de base, au jour 30, au jour 60 et au jour 90, et elles ont été analysées en utilisant un dispositif de tomographie aux rayons X assistée par ordinateur (Quantum FX Caliper^{®}, Life Sciences, Perkin Elmer, Waltham, MA) de la PIV Platform, EA2496, Montrouge, France.

La source de rayons X a été réglée à 90kV et 160µA.

Les images tridimensionnelles ont été acquises avec taille de voxel isotrope de 20µm.

Un fantôme de densité interne, calibré en mg d'hydroxyapatite, a été utilisé pour graduer la densité osseuse.

Les données brutes de haute résolution 3D ont été obtenues par une rotation de 360°, à la fois de la source de rayons X et du détecteur à panneau plat, autour de l'échantillon (durée de balayage de 3 min).

Les rendus tridimensionnels ont été ensuite extraits des cadres de données Dico en utilisant le logiciel d'imagerie OsiriX^{®} (b5.7.1, distribué sous licence LGPL, Dr A. Rosset, Genève, Suisse).

La quantification de l'os régénéré à l'intérieur de chaque défaut a été effectuée en utilisant le logiciel CTscan Analyzer^{®} (Skyscan, version 1.13.5.1, Kontic, Belgique).

Un volume global d'intérêt (VOI) a été tracé par interpolation de la région 2D d'intérêt sur des sections consécutives pour isoler la zone du défaut initial.

Le VOI interpolé obtenu comprenait seulement la zone de défaut d'os remodelé.

Un seuil global a été déterminé de façon interactive pour la sélection d'os et pour éliminer le bruit de fond.

Les fractions volumétriques de « nouveau tissu » ont été exprimées relativement au volume total du défaut initial ;

### Statistiques

Les résultats dans chaque groupe ont été exprimés comme la valeur moyenne ± l'écart type. Un test de Fischer est utilisé pour discriminer statistiquement les groupes biologiques.

### Résultats

La figure 4 montre sous forme de graphe les résultats de viabilité cellulaire des ostéoblastes primaires de rat en présence d'un contrôle (100%), de BG-PCL et de BG-PCL-Fis.

La mesure de la viabilité de l'activité cellulaire est basée sur l'activité XTT après 7 jours de culture.

Sur cette figure, l'activité mitochondriale est exprimée en tant que pourcentage de l'état du contrôle (CTRL) .

Comme on peut le voir en figure 4, aucune différence significative sur la viabilité cellulaire n'est mise en évidence entre le contrôle et l'implant selon l'invention ou selon l'art antérieur.

Cette absence de différence significative démontre l'absence d'effets adverses des produits de dissolution de BG-PCL et de BG-PCL-Fis sur la croissance et la prolifération cellulaire.

Le comportement des ostéoblastes primaires sur des tranches d'os cortical humain, de disques de BG-PCL de l'exemple 1, et de disques De BG-PCL Fis de l'exemple 2, a été étudié par microscopie électronique à balayage.

Les tranches d'os cortical humain ont été utilisées en tant que contrôle.

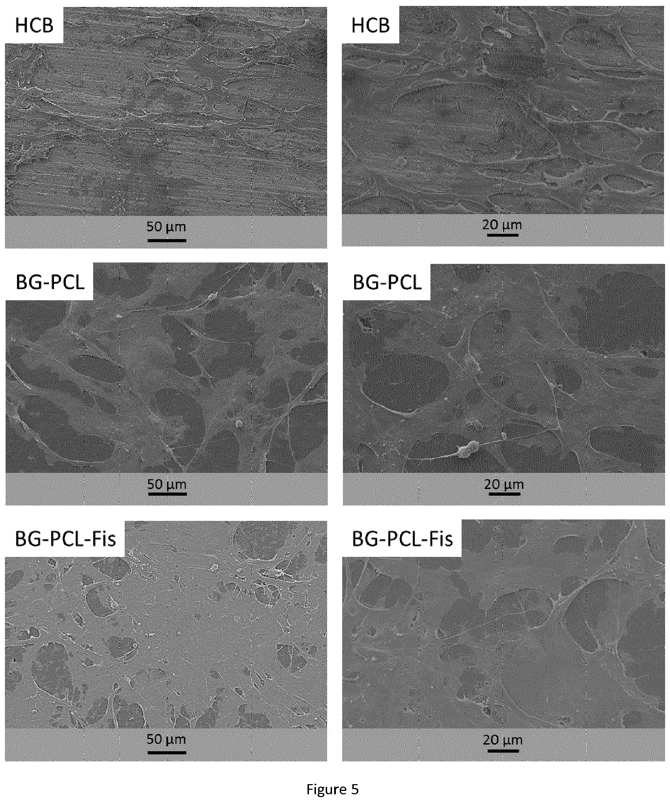

La figure 5 montre les images de microscopie électronique à balayage de RPO cultivés sur :
- Photographies du haut : les tranches d'os cortical humain à 2 grandissements différents,
- Photographies du milieu : les disques de BG-PCL,
- Photographies du bas : les disques de BG-PCL-Fis.

Comme on peut le voir en figure 5, dans tous les cas, les cellules couvraient la surface des matériaux, présentaient des formes en étoile et des filopodes connectés, démontrant ainsi une adhésion cellulaire appropriée.

A noter que les cellules semblent s'étaler plus efficacement à la surface de BG-PCL et encore plus à la surface de BG-PCL-Fis, par rapport à l'os cortical humain, ce qui montre la création d'un environnement favorisant l'adhésion cellulaire du matériau hybride dopé de l'invention.

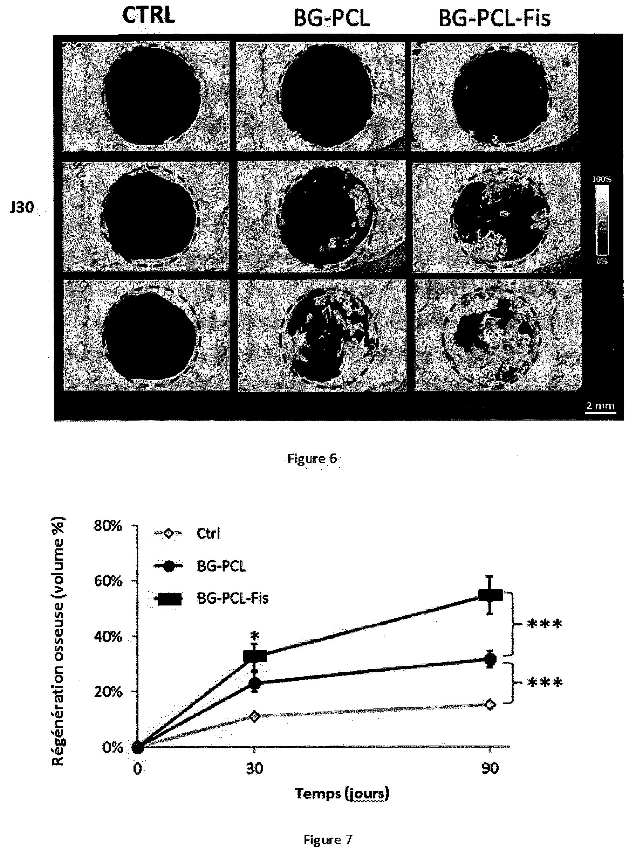

De façon encore plus importante, les images de tomographie assistée par ordinateur reproduites en figure 6 montrent qu'après 30 jours :
- Les défauts vides (contrôle laissés vides) présentent une faible régénération osseuse d'environ 10% (régénération spontanée insuffisante, définissant le caractère critique du défaut),
- Les défauts remplis avec BG-PCL présentent un volume d'os significativement plus élevé d'approximativement 30%, et
- Les défauts remplis avec BG-PCL-Fis atteignent une régénération osseuse de 55%.

A la fin des 90 jours d'essai, le volume osseux le volume osseux atteignait environ 15% chez les contrôle alors que plus de 30% des défauts osseux sont réparés en employant des implants en BG-PCL.

De façon remarquable, en employant l'implant dopé à la fisétine BG-PCL-Fis de l'exemple 2, la formation d'os nouveau s'étendait au défaut entier et couvrait plus de 55% du défaut initial.

Ces résultats sont montrés sous forme de courbes en figure 7.

## Revendications

1. Matériau d'implant pour le comblement de défauts osseux, la régénération osseuse et l'ingénierie tissulaire de l'os,
**caractérisé en ce que**
il comprend un matériau hybride comprenant :
• un verre bioactif M à base de SiO₂ et CaO, optionnellement contenant P₂O₅ et/ou optionnellement dopé au strontium, et
• un polymère biodégradable P est choisi parmi :
- les polysaccharides biorésorbables, de préférence choisis parmi le dextran, l'acide hyaluronique, l'agar, le chitosane, l'acide alginique, l'alginate de sodium ou de potassium, le galactomannane, le carraghénane, la pectine,
- les polyesters biorésorbables, de préférence l'alcool polyvinylique ou le poly(acide lactique), et
- les polymères synthétiques biodégradables, de préférence un polyéthylène glycol, le poly(caprolactone),
**et en ce que** ce matériau hybride est dopé avec un nutriment ostéoinducteur N choisi parmi la vitamine D2 (ergocalciférol), la vitamine D3 (cholicalciférol), la vitamine K1, la vitamine K2, les acides gras oméga-3, l'acide punicique, l'acide α-lipoïque, les anthocyanines, les flavonols, les procyanidines, le tyrosol, l'oleuropéïne, la naringénine, la punicalagine, l'acide ellagique et la phycocyanine.

2. Matériau d'implant pour le comblement de défauts osseux, la régénération osseuse et l'ingénierie tissulaire de l'os selon la revendication 1 **caractérisé en ce que** le matériau hybride dopé en un nutriment ostéoinducteur comprend 30% en masse de verre bioactif M à base de SiO₂ et CaO, par rapport à la masse totale (verre bioactif M + polymère biodégradable P + nutriment ostéoinducteur N), 69% en masse de poly(caprolactone), par rapport à la masse totale (verre bioactif M + polymère biodégradable P + nutriment ostéoinducteur N), et 1% en masse de fisétine et/ou d'hydroxytyrosol, par rapport à la masse totale (verre bioactif M + polymère biodégradable P + nutriment ostéoinducteur N)

3. Matériau d'implant pour le comblement de défauts osseux, la régénération osseuse et l'ingénierie tissulaire de l'os selon la revendication 1 **caractérisé en ce que** le matériau hybride dopé en un nutriment ostéinducteur comprend 40% en masse de verre bioactif M à base de SiO₂ et CaO, , par rapport à la masse totale(verre bioactif M + polymère biodégradable P + nutriment ostéoinducteur N), 59% en masse de poly(caprolactone), par rapport à la masse totale(verre bioactif M + polymère biodégradable P + nutriment ostéoinducteur N), et 1% en masse de fisétine et/ou d'hydroxytyrosol, par rapport à la masse totale (verre bioactif M + polymère biodégradable P + nutriment ostéoinducteur N).

4. Implant en un matériau hybride pour le comblement de défauts osseux, la régénération osseuse et l'ingénierie tissulaire de l'os, **caractérisé en ce qu'**il comprend un matériau selon l'une quelconque des revendications précédentes.

5. Procédé de fabrication d'un implant en un matériau hybride pour le comblement de défauts osseux, la régénération osseuse et l'ingénierie tissulaire de l'os, **caractérisé en ce qu'**il comprend les étapes suivantes :
a) sélection d'un verre bioactif M à base de SiO₂ et CaO, contenant optionnellement P₂O₅ et/ou optionnellement dopé au strontium,
b) sélection d'un polymère biodégradable P qui est soluble dans au moins un solvant S1 et insoluble dans au moins un solvant S, choisi parmi :
- les polysaccharides biorésorbables, de préférence choisis parmi le dextran, l'acide hyaluronique, l'agar, le chitosane, l'acide alginique, l'alginate de sodium ou de potassium, le galactomannane, le carraghénane, la pectine,
- les polyesters biorésorbables de préférence l'alcool polyvinylique ou le poly(acide lactique), et
- les polymères synthétiques biodégradables, de préférence un polyéthylène glycol, ou le poly(caprolactone)
c) sélection de microsphères d'un agent porogène A ayant des diamètres et des tailles correspondant aux diamètres et tailles recherchés des pores dans le matériau constituant l'implant à fabriquer, cet agent porogène A étant :
- en un polymère insoluble dans le au moins un solvant S1 et soluble dans le au moins un solvant S,
d) sélection d'au moins un nutriment ostéoinducteur N :
- soluble dans au moins un solvant S2 identique ou différent du solvant S1 mais miscible avec le solvant S1,qui ne dégrade pas le polymère biodégradable P ,qui ne dégrade pas le verre bioactif M, et dans lequel les microsphères d'agent porogène A ne sont pas solubles, et
- insoluble dans le solvant S,
- choisi parmi la vitamine D2 (ergocalciférol), la vitamine D3 (cholicalciférol) et la vitamine K1, la vitamine K2, les acides gras oméga-3, l'acide punicique, l'acide α-lipoïque, les anthocyanines, les flavonols, les procyanidines, le tyrosol, l'oleuropéïne, la naringénine, la punicalagine, l'acide ellagique et la phycocyanine,
e) introduction des microsphères de l'agent porogène A dans un moule ayant la forme et la taille recherchées pour l'implant, ces microsphères formant un empilement compact correspondant à la forme et à la taille des pores à obtenir dans le matériau d'implant, et représentant au moins au moins 60% en volume, de préférence au moins 70% en volume par rapport au volume total du mélange agent porogène A-polymère biodégradable P-précurseurs alcoxydes du verre bioactif M-nutriment ostéoinducteur N,
f) introduction du nutriment ostéoinducteur N, en solution dans le solvant S2, dans le polymère biodégradable P en solution dans le solvant S1, et mélange,
g) introduction du mélange obtenu à l'étape f) dans les précurseurs alcoxydes du verre bioactif M,
h) introduction du mélange obtenu à l'étape g) dans le moule,
i) solidification du mélange contenu dans le moule après l'étape h),
j) démoulage du mélange obtenu à l'étape i),
k) élimination des microsphères d'agent porogène A par lavage avec le solvant S.

6. Procédé selon la revendication 5,
**caractérisé en ce que** le matériau de l'agent porogène A est choisi parmi les polymères biodégradables insolubles dans le au moins un solvant S1 et le au moins un solvant S2 et solubles dans le au moins un solvant S, de préférence choisi parmi les polyméthacrylates d'alkyle en C₁ à C₄, de préférence le polyméthacrylate de méthyle ou le polyméthacrylate de butyle, le polyuréthane, l'acide polyglycolique, les différentes formes d'acides polylactiques, les copolymères d'acides lactique-coglycoliques, le poly(caprolactone), le polypropylène fumarate, la paraffine et le naphtalène, ou l'acrylonitrile butadiène styrène (ABS),
le matériau de l'agent porogène A étant différent du polymère biodégradable P.

7. Procédé selon la revendication 5 ou 6, **caractérisé en ce que** le rapport en poids polymère biodégradable P/ verre bioactif M est compris entre 20/80 et 80/20, bornes incluses et ce que le nutriment ostéoinducteur N est présent en une quantité comprise entre 0,1 et 10%, de préférence entre 0,1% et 5%, plus préférablement de 1%, en masse par rapport à la masse totale du matériau obtenu à l'étape k).

8. Procédé selon l'une quelconque des revendications 5 à 7 **caractérisé en ce que** :
- le verre bioactif M est un verre à base de SiO₂ et de CaO,
- le polymère biodégradable P est le poly(caprolactone),
- le nutriment ostéoinducteur N est la fisétine et/ou l'hydroxytyrosol,
- le matériau des microsphères d'agent porogène A est la paraffine,
- le solvant S est le cyclohexane,
- le solvant S1 est identique au solvant S2 et est le tétrahydrofurane (THF).

## Patentansprüche

1. Implantatmaterial für die Auffüllung von Knochendefekten, die Knochenregeneration und die Knochengewebezüchtung,
**dadurch gekennzeichnet, dass**
es ein Hybridmaterial umfasst, das Folgendes umfasst:
• ein bioaktives Glas M auf Basis von SiO₂ und CaO, das wahlweise P2O5 enthält und/oder wahlweise mit Strontium dopiert ist, und
• ein biologisch abbaubares Polymer P, das ausgewählt ist aus:
- biologisch resorbierbaren Polysacchariden, vorzugsweise ausgewählt aus Dextran, Hyaluronsäure, Agar, Chitosan, Alginsäure, Natrium- oder Kaliumalginat, Galactomannan, Carrageen, Pektin,
- biologisch resorbierbare Polyestern, vorzugsweise Polyvinylalkohol oder Polymilchsäure, und
- biologisch abbaubaren synthetischen Polymeren, vorzugsweise einem Polyethylenglykol, Polycaprolacton,
**und dadurch, dass** dieses Hybridmaterial mit einem osteoinduktiven Nährstoff N dopiert ist, der aus Vitamin D2 (Ergocalciferol), Vitamin D3 (Cholicalciferol), Vitamin K1, Vitamin K2, Omega-3-Fettsäuren, Punicinsäure, α-Liponsäure, Anthocyaninen, Flavonolen, Procyanidinen, Tyrosol, Oleuropein, Naringenin, Punicalagin, Ellagsäure und Phycocyanin ausgewählt ist.

2. Implantatmaterial für die Auffüllung von Knochendefekten, die Knochenregeneration und die Knochengewebezüchtung nach Anspruch 1, **dadurch gekennzeichnet, dass** das Hybridmaterial, das mit einem osteoinduktiven Nährstoff dopiert ist, in Bezug auf die Gesamtmasse (bioaktives Glas M + biologisch abbaubares Polymer P + osteoinduktiver Nährstoff N) 30 Massen-% bioaktives Glas M auf Basis von SiO₂ und CaO, in Bezug auf die Gesamtmasse (bioaktives Glas M + biologisch abbaubares Polymer P + osteoinduktiver Nährstoff N) 69 Massen-% Polycaprolacton, und in Bezug auf die Gesamtmasse (bioaktives Glas M + biologisch abbaubares Polymer P + osteoinduktiver Nährstoff N) 1% Massen-% Fisetin und/oder Hydroxytyrosol umfasst.

3. Implantatmaterial für die Auffüllung von Knochendefekten, die Knochenregeneration und die Knochengewebezüchtung nach Anspruch 1, **dadurch gekennzeichnet, dass** das Hybridmaterial, das mit einem osteoinduktiven Nährstoff dopiert ist, in Bezug auf die Gesamtmasse (bioaktives Glas M + biologisch abbaubares Polymer P + osteoinduktiver Nährstoff N) 40 Massen-% bioaktives Glas M auf Basis von SiO₂ und CaO, in Bezug auf die Gesamtmasse (bioaktives Glas M + biologisch abbaubares Polymer P + osteoinduktiver Nährstoff N) 59 Massen-% Polycaprolacton, und in Bezug auf die Gesamtmasse (bioaktives Glas M + biologisch abbaubares Polymer P + osteoinduktiver Nährstoff N) 1% Massen-% Fisetin und/oder Hydroxytyrosol umfasst.

4. Implantat aus einem Hybridmaterial für die Auffüllung von Knochendefekten, die Knochenregeneration und die Knochengewebezüchtung, **dadurch gekennzeichnet, dass** es ein Material nach einem der vorstehenden Ansprüche umfasst.

5. Verfahren zur Herstellung eines Implantats aus einem Hybridmaterial für die Auffüllung von Knochendefekten, die Knochenregeneration und die Knochengewebezüchtung, **dadurch gekennzeichnet, dass** es die folgenden Schritte umfasst:
a) Auswahl eines bioaktiven Glases M auf Basis von SiO₂ und CaO, das wahlweise P₂O₅ enthält und/oder wahlweise mit Strontium dopiert ist,
b) Auswahl eines biologisch abbaubaren Polymers P, das in mindestens einem Lösemittel S1 löslich und in mindestens einem Lösemittel S unlöslich ist, ausgewählt aus:
- biologisch resorbierbaren Polysacchariden, vorzugsweise ausgewählt aus Dextran, Hyaluronsäure, Agar, Chitosan, Alginsäure, Natrium- oder Kaliumalginat, Galactomannan, Carrageen, Pektin,
- biologisch resorbierbaren Polyestern, vorzugsweise Polyvinylalkohol oder Polymilchsäure, und
- biologisch abbaubaren synthetischen Polymeren, vorzugsweise einem Polyethylenglykol oder Polycaprolacton
c) Auswahl von Mikrosphären eines Porenbildners A, die Durchmesser und Größen aufweisen, die den angestrebten Durchmessern und Größen der Poren im Material entsprechen, das das herzustellende Implantat konstituiert, wobei dieser Porenbildner A:
- in einem Polymer vorliegt, das in dem mindestens einen Lösemittel S1 unlöslich ist und in dem mindestens einen Lösemittel S löslich ist,
d) Auswahl mindestens eines osteoinduktiven Nährstoffs N:
- der in mindestens einem Lösemittel S2 löslich ist, das mit dem Lösemittel S1 identisch ist oder sich von diesem unterscheidet, jedoch mit dem Lösemittel S1 mischbar ist, der das biologisch abbaubare Polymer P nicht abbaut, der das biologisch abbaubare Glas M nicht abbaut und in dem die Mikrosphären des Porenbildners A nicht löslich sind, und
- der im Lösemittel S unlöslich ist,
- ausgewählt aus Vitamin D2 (Ergocalciferol), Vitamin D3 (Cholicalciferol) und Vitamin K1, Vitamin K2, Omega-3-Fettsäuren, Punicinsäure, α-Liponsäure, Anthocyaninen, Flavonolen, Procyanidinen, Tyrosol, Oleuropein, Naringenin, Punicalagin, Ellagsäure und Phycocyanin,
e) Einführung der Mikrosphären des Porenbildners A in eine Gussform, die die angestrebte Form und Größe für das Implantat aufweist, wobei diese Mikrosphären eine kompakte Packung bilden, die der Form und der Größe der Poren entspricht, die im Implantatmaterial zu erhalten sind, und in Bezug auf das Gesamtvolumen des Gemischs von Porenbildner A, biologisch abbaubarem Polymer P, Alkoxidvorläufern des bioaktiven Glases M, osteoinduktivem Nährstoff N mindestens 60 Volumen-%, vorzugsweise 70 Volumen-% darstellt,
f) Einführung des osteoinduktiven Nährstoffs N, in Lösung im Lösemittel S2, in das biologisch abbaubare Polymer P in Lösung im Lösemittel S1 und Gemisch,
g) Einführung des im Schritt f) erhaltenen Gemischs in die Alkoxidvorläufer des bioaktiven Glases M,
h) Einführung des im Schritt g) erhaltenen Gemischs in die Gussform,
i) Verfestigung des in der Gussform nach dem Schritt h) enthaltenen Gemischs,
j) Entnahme des im Schritt i) erhaltenen Gemischs aus der Gussform,
k) Eliminierung der Mikrosphären von Porenbildner A durch Spülung mit dem Lösemittel S.

6. Verfahren nach Anspruch 5,
**dadurch gekennzeichnet, dass** das Material des Porenbildners A aus biologisch abbaubaren Polymeren ausgewählt ist, die in dem mindestens einen Lösemittel S1 und dem mindestens einen Lösemittel S2 unlöslich sind und in dem mindestens einen Lösemittel S löslich sind, vorzugsweise ausgewählt aus Alkylpolymethacrylaten mit C₁ bis C₄, vorzugsweise Methylpolymethacrylat oder Butylpolymethacrylat, Polyurethan, Polyglycolsäure, unterschiedlichen Formen von Polymilchsäuren, Milchsäure-Coglycolsäure-Copolymeren, Polycaprolacton, Fumaratpolypropylen, Paraffin und Naphtalen, oder Acrylonitril-Butadien-Styrol (ABS),
wobei sich der Porenbildner A von dem biologisch abbaubaren Polymer P unterscheidet.

7. Verfahren nach Anspruch 5 oder 6, **dadurch gekennzeichnet, dass** das Gewichtsverhältnis von biologisch abbaubarem Polymer P / bioaktivem Glas M zwischen 20/80 und 80/20, Grenzwerte inbegriffen, beträgt, und dadurch, dass der osteoinduktive Nährstoff N in Bezug auf die Gesamtmenge des im Schritt k) erhaltenen Materials in einer Menge zwischen 0,1 und 10%, vorzugsweise zwischen 0,1% und 5%, bevorzugter von 1% vorliegt.

8. Verfahren nach einem der Ansprüche 5 bis 7, **dadurch gekennzeichnet, dass**:
- das bioaktive Glas M ein Glas auf Basis von SiO₂ und von CaO ist,
- das biologisch abbaubare Polymer P Polycaprolacton ist,
- der osteoinduktive Nährstoff N Fisetin und/oder Hydroxytyrosol ist,
- das Material der Mikrosphären von Porenbildner A Paraffin ist,
- das Lösemittel S Cyclohexan ist,
- das Lösemittel S1 mit dem Lösemittel S2 identisch ist und Tetrahydrofuran (THF) ist.

## Claims

1. Implant material for filling in bone defects, bone regeneration and bone tissue engineering,
**characterised in that**
it comprises a hybrid material comprising:
• a bioactive glass M made from SiO₂ and CaO, optionally containing P₂O₅ and/or optionally doped with strontium, and
• a biodegradable polymer P chosen from:
- bioabsorbable polysaccharides, preferably chosen from dextran, hyaluronic acid, agar, chitosan, alginic acid, sodium or potassium alginate, galactomannan, carageenan, pectin,
- bioabsorbable polyesters, preferably polyvinyl alcohol or poly(lactic acid), and
- biodegradable synthetic polymers, preferably a polyethylene glycol, poly(caprolactone),
**and in that** this hybrid material is doped with an osteoinductive nutrient N chosen from vitamin D2 (ergocalciferol), vitamin D3 (cholicalciferol), vitamin K1, vitamin K2, omega-3 fatty acids, punicic acid, α-lipoic acid, anthocyanins, flavonols, procyanidins, tyrosol, oleuropein, naringenin, punicalagin, ellagic acid and phycocyanin.

2. Implant material for filling in bone defects, bone regeneration and bone tissue engineering according to claim 1 **characterised in that** the hybrid material doped with an osteoinductive nutrient comprises 30% by weight of bioactive glass M made from SiO₂ and CaO, with respect to the total weight (bioactive glass M + biodegradable polymer P + osteoinductive nutrient N), 69% by weight of poly(caprolactone), with respect to the total weight (bioactive glass M + biodegradable polymer P + osteoinductive nutrient N), and 1% by weight of fisetin and/or hydroxytyrosol, with respect to the total weight (bioactive glass M + biodegradable polymer P + osteoinductive nutrient N).

3. Implant material for filling in bone defects, bone regeneration and bone tissue engineering according to claim 1 **characterised in that** the hybrid material doped with an osteoinductive nutrient comprises 40% by weight of bioactive glass M made from SiO₂ and CaO, with respect to the total weight (bioactive glass M + biodegradable polymer P + osteoinductive nutrient N), 59% by weight of poly(caprolactone), with respect to the total weight (bioactive glass M + biodegradable polymer P + osteoinductive nutrient N), and 1% by weight of fisetin and/or hydroxytyrosol, with respect to the total weight (bioactive glass M + biodegradable polymer P + osteoinductive nutrient N).

4. Implant material for filling in bone defects, bone regeneration and bone tissue engineering, **characterised in that** it comprises a material according to any of the preceding claims.

5. Method for manufacturing an implant made of a hybrid material for filling in bone defects, bone regeneration and bone tissue engineering, **characterised in that** it comprises the following steps:
a) selecting a bioactive glass made from SiO₂ and CaO, optionally containing P₂O₅ and/or optionally doped with strontium,
b) selecting a biodegradable polymer P that is soluble in at least one solvent S1 and insoluble in at least one solvent S, chosen from:
- bioabsorbable polysaccharides, preferably chosen from dextran, hyaluronic acid, agar, chitosan, alginic acid, sodium or potassium alginate, galactomannan, carageenan, pectin,
- bioabsorbable polyesters preferably polyvinyl alcohol or poly(lactic acid), and
- biodegradable synthetic polymers, preferably a polyethylene glycol or poly(caprolactone),
c) selecting microspheres of a blowing agent A having diameters and sizes that correspond to the sought diameters and sizes of the pores in the material forming the implant to be manufactured, this blowing agent A being:
- made of a polymer insoluble in the at least one solvent S1 and soluble in the at least one solvent S,
d) selecting at least one osteoinductive nutrient N:
- soluble in at least one solvent S2 identical or different from the solvent S1 but miscible with the solvent S1, which does not degrade the biodegradable polymer P, which does not degrade the bioactive glass M, and wherein the microspheres of blowing agent A are not soluble, and
- insoluble in the solvent S,
- and **in that** this hybrid material is doped with an osteoinductive nutrient N chosen from vitamin D2 (ergocalciferol), vitamin D3 (cholicalciferol), vitamin K1, vitamin K2, omega-3 fatty acids, punicic acid, α-lipoic acid, anthocyanins, flavonols, procyanidins, tyrosol, oleuropein, naringenin, punicalagin, ellagic acid and phycocyanin,
e) introducing microspheres of the blowing agent A in a mould having the shape and the size sought for the implant, these microspheres forming a compact stack corresponding to the shape and size of the pores to be obtained in the implant material, and representing at least 60% by volume, preferably at least 70% by volume, with respect to the total volume of the blowing agent A- biodegradable polymer P-alkoxide precursors of the bioactive glass M-osteoinductive nutrient N mixture,
f) introducing the osteoinductive nutrient N, in solution in the solvent S2, in the biodegradable polymer P in solution in the solvent S1, and mixture,
g) introducing of the mixture obtained in step f) into the alkoxide precursors of the bioactive glass M,
h) introducing of the mixture obtained in step g) into the mould,
i) solidifying the mixture contained in the mould after step h),
j) demoulding of the mixture obtained in step i),
k) eliminating microspheres of blowing agent A by washing with the solvent S.

6. Method according to claim 5,
**characterised in that** the material of the blowing agent A is chosen from biodegradable polymers insoluble in the at least one solvent S1 and the at least one solvent S2 and soluble in the at least one solvent S, preferably chosen from C₁ to C₄ polyalkylmethacrylate, preferably polymethyl methacrylate or poly(butyl methacrylate), polyurethane, polyglycolic acid, the different forms of polylactic acids, copolymers of lactic-co-glycolic acid, poly(caprolactone), le polypropylene fumarate, paraffin and naphthalene, or acrylonitrile-butadienestyrene (ABS),
the material of the blowing agent A being different from the biodegradable polymer P.

7. Method according to claim 5 or 6, **characterised in that** the biodegradable polymer P/ bioactive glass M ratio by weight is comprised between 20/80 and 80/20, limits included and **in that** the osteoinductive nutrient N is present in a quantity comprised between 0.1 and 10%, preferably between 0.1% and 5%, more preferably 1%, by weight with respect to the total weight of the material obtained in step k).

8. Method according to any of claims 5 to 7 **characterised in that**:
- the bioactive glass M is a glass made from SiO₂ and CaO,
- the biodegradable polymer P is poly(caprolactone),
- the osteoinductive nutrient N is fisetin and/or hydroxytyrosol,
- the material of the microspheres of blowing agent A is paraffin,
- the solvent S is cyclohexane,
- the solvent S1 is identical to the solvent S2 and is tetrahydrofuran (THF).
